# EUROPEAN PATENT APPLICATION

(11) **EP 4 629 501 A1**
(43) Date of publication of application: **08.10.2025**
(21) Application number: 23897556.9
(22) Date of filing: 17.11.2023
(51) Int. Cl.: H02N 2/12

(54) **VIBRATION TYPE ACTUATOR AND MEDICAL DEVICE AND ELECTRONIC DEVICE HAVING SAME**

(30) Priority: 29.11.2022 JP 2022190041
(71) Applicant: CANON KABUSHIKI KAISHA, Tokyo 146-8501 (JP)
(72) Inventor: TSUCHIYA, Satoshi, Tokyo 146-8501 (JP); KITAJIMA, Satoru, Tokyo 146-8501 (JP)
(74) Representative: Canon Europe Limited
(86) International application number: PCT/JP2023/041485
(87) International publication number: WO 2024/116905

(57) **Abstract**

There is provided a vibration actuator that includes a vibration body having an electro-mechanical energy conversion element and an elastic body, and a contact body in contact with the elastic body. The vibration body and the contact body move relatively to each other due to vibration of the vibration body. At least one of the elastic body and the contact body has a base material made of an austenitic steel material. A portion of a surface of the base material includes a first surface having higher contents [mass%] of nickel and phosphorus than the base material, and a second surface having a higher content [mass%] of nitrogen than the base material. The elastic body and the contact body are in contact with each other on the second surface.

## Description

### Technical Field

The present invention relates to a vibration actuator, and a medical device and an electronic device having the same.

### Background Art

A vibration actuator does not require magnetism as a principle of driving because it excites a vibration body to vibrate by electro-mechanical energy conversion (for example, a piezoelectric element) and drives a contact body that is in frictional contact with the vibration body by frictional force. For this reason, a vibration actuator can be made of a non-magnetic material, and is less likely to affect external magnetic fields and is less susceptible to the influence of external magnetic fields. Therefore, such a vibration actuator can be used together with an electron beam lithography apparatus that uses a weak magnetic field or a magnetic resonance imaging apparatus (hereinafter, MRI apparatus) that operates under a strong magnetic field environment.

On the other hand, in order to improve the durability of a vibration actuator, there have been discussed technologies for improving wear resistance by using a material with high hardness for the contact surface of a moving body (contact body) that comes into contact with the vibration body. Patent Document 1 discusses a vibrating elastic body or moving body, which is made of a single stainless steel material and subjected to nitriding treatment, that constitutes a vibrational wave driving device.

Patent Document 2 discusses a vibration actuator in which a plating film is formed on the surface of an elastic base material made of an iron-based metal, and after the plating process, the upper surface of the base material, which is to be the surface of friction with a moving body in the elastic body, is nitrided to form a nitrided layer. Both technologies are intended to improve wear resistance.

According to the technology discussed in Patent Document 1, non-magnetic austenitic stainless steel (SUS304 or the like) is used as the base material of the elastic body that constitutes the vibration body. However, even if the stainless steel is austenitic, the nitrided layer formed on the surface by nitriding treatment is magnetic, so there is an issue that the vibration body is not sufficiently demagnetized.

According to the technique discussed in Patent Document 2, a plating film is formed as an anti-nitriding mask to prevent the formation of a nitrided layer before the nitriding treatment of the elastic body. However, there is an issue in that the plating film is not sufficiently demagnetized.

### Citation List

### Patent Literature

PTL 1: Japanese Patent Application Laid-Open No. 2024-80947
PTL 2: Japanese Patent No. 6292955

### Summary of Invention

### Technical Problem

In view of the above, the present invention is directed to providing a vibration actuator including an elastic body that is both wear-resistant and non-magnetic.

### Solution to Problem

In order to solve the above problems, a vibration actuator includes a vibration body having an electro-mechanical energy conversion element and an elastic body, and a contact body in contact with the elastic body, wherein the vibration body and the contact body move relatively to each other due to vibration of the vibration body, wherein at least one of the elastic body and the contact body has a base material made of an austenitic steel material, wherein a portion of a surface of the base material includes a first surface having higher contents [mass%] of nickel and phosphorus than a part of the base material other than the surface, and a second surface having a higher content [mass%] of nitrogen than the part of the base material other than the surface, and wherein the elastic body and the contact body are in contact with each other on the second surface.

### Advantageous Effects of Invention

According to the above invention, it is possible to provide a vibration actuator including an elastic body that is both wear-resistant and non-magnetic.

### Brief Description of Drawings

[Fig. 1] Fig. 1is a schematic cross-sectional view of a configuration of a vibration actuator according to a first exemplary embodiment of the present invention.
[Fig. 2] Fig. 2 is a schematic perspective view of a configuration of an elastic body in Fig. 1.
[Fig. 3A] Fig. 3A is a schematic front view of a configuration of the elastic body in Fig. 1.
[Fig. 3B] Fig. 3B is a schematic cross-sectional view of a configuration of the elastic body in Fig. 1.
[Fig. 4] Fig. 4 is a schematic cross-sectional view of a configuration of materials of the elastic body in Fig. 1.
[Fig. 5] Fig. 5 is a diagram illustrating a manufacturing process of the elastic body in Fig. 1.
[Fig. 6] Fig. 6 is a schematic diagram illustrating a method for evaluating the magnetic property of a material according to a first exemplary embodiment of the present invention.
[Fig. 7] Fig. 7 is a table illustrating the evaluation results of magnetic properties of materials according to the first exemplary embodiment of the present invention.
[Fig. 8] Fig. 8 is a graph illustrating the evaluation results of magnetic properties of the materials according to the first exemplary embodiment of the present invention.
[Fig. 9A] Fig. 9A is a graph illustrating the results of elemental analysis of the materials according to the first exemplary embodiment of the present invention.
[Fig. 9B] Fig. 9B is a graph illustrating the results of elemental analysis of the materials according to the first exemplary embodiment of the present invention.
[Fig. 10] Fig. 10 is a schematic exploded perspective view of a configuration of a vibration actuator according to a second exemplary embodiment of the present invention.
[Fig. 11] Fig. 11 is a schematic perspective view of a configuration of a magnetic resonance imaging (MRI) diagnostic apparatus equipped with a vibration actuator according to an exemplary embodiment of the present invention.

### Description of Embodiments

Hereinafter, exemplary embodiments of the present invention will be described in detail with reference to the accompanying drawings.

### First Exemplary Embodiment

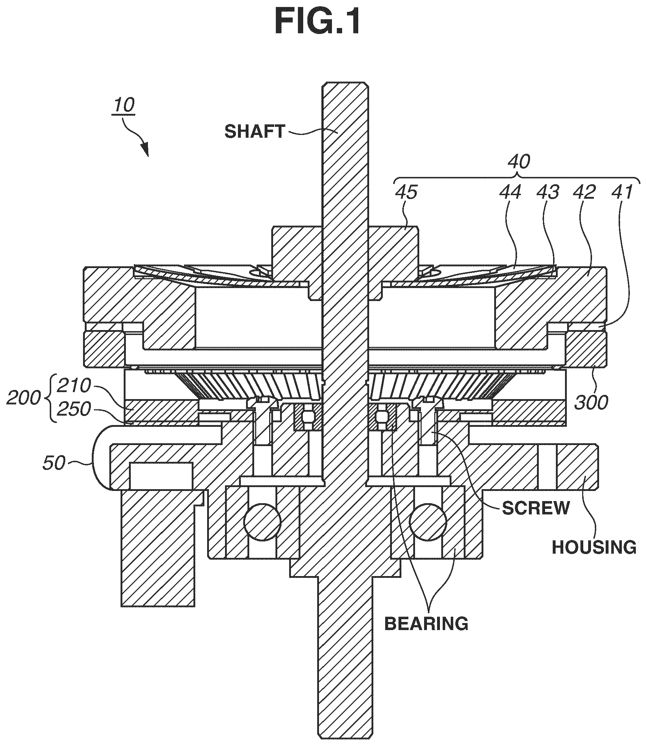

Fig. 1 is a schematic cross-sectional view of a configuration of a vibration actuator 10 according to a first exemplary embodiment of the present invention. In the vibration actuator 10, the mechanical configuration of a vibration body 200, a contact body 300 (moving body, driven body), a pressure mechanism 40, and the like is functionally equivalent to that of the vibration actuator discussed in Japanese Patent Application Laid-Open No. 2017-108615, for example.

The above term "contact body" refers to a member that comes into contact with the vibration body and moves relative to the vibration body due to vibrations generated in the vibration body. The contact between the contact body and the vibration body is not limited to direct contact without any other member interposed between the contact body and the vibration body. The contact between the contact body and the vibration body may be indirect contact with another member interposed between the contact body and the vibration body, as long as the contact body moves relative to the vibration body due to vibrations generated in the vibration body. The "other/another member" is not limited to a member independent of the contact body and the vibration body (for example, a high-friction material made of a sintered body). The "other/another member" may be a surface-treated portion formed on the contact body or the vibration body by plating, nitriding, or the like.

Referring to Fig. 1, the vibration actuator 10 includes the vibration body 200 formed in an annular shape, the contact body 300 formed in an annular shape, and the pressure mechanism 40. The vibration actuator 10 also includes a shaft, a housing, bearings, and screws. These are mainly made of austenitic steel materials, brass, phosphor bronze, aluminum alloys, ceramics, and other non-magnetic materials. Austenitic steel materials are steel materials mainly made of austenitic structures, such as austenitic stainless steel and austenitic heat-resistant steel.

The vibration body 200 has an elastic body 210, a piezoelectric element 250 that is an electro-mechanical energy conversion element joined to the elastic body 210, and a power supply member 50 that is joined to the piezoelectric element 250 and applies a driving voltage which is an alternating-current voltage to the piezoelectric element 250.

The contact body 300 has a main body formed in an annular shape, and a contact spring. The contact body is made of a non-magnetic material such as an austenitic steel material or an aluminum alloy. The contact surface of the contact body 300 to the vibration body 200 is subjected to hardening treatment such as carburizing, nitriding, and alumite treatment to improve wear resistance. The contact body 300 does not have to be completely non-magnetic, and may be partially made of a ferromagnetic material as long as its volume ratio is small. For example, as in the contact body discussed in Japanese Patent Application Laid-Open No. 2022-29751, an aluminum alloy, which is a non-magnetic material, may be used for the main body, and a quenched martensitic stainless steel, which is a ferromagnetic material, may be used for a part near the contact surface.

The pressure mechanism 40 has a vibration-damping rubber 41, a pressure spring receiving member 42, a pressure spring receiving rubber 43, a pressure spring 44, and a pressure spring fixing member 45. The vibration body 200 and the contact body 300 are arranged concentrically with the shaft as the central axis, and are in pressure contact (frictional contact) with each other in the thrust direction of the shaft by the pressure mechanism 40 fixed to the shaft. Specifically, the pressure spring 44 whose movement is restricted by the pressure spring fixing member 45 fixed to the shaft, presses the contact body 300 in the thrust direction via the vibration-damping rubber 41, the pressure spring receiving member 42, and the pressure spring receiving rubber 43. With this configuration, the contact body 300 and the vibration body 200 are in stable contact with each other.

In the vibration actuator 10, a driving voltage, which is an alternating-current voltage, is applied to the piezoelectric element 250 through the power supply member 50 to excite driving vibration in the vibration body 200. Although the form of the driving vibration depends on the number and arrangement of a plurality of electrodes of the piezoelectric element 250, the piezoelectric element 250 is designed such that the excited driving vibration constitutes an n-th order traveling wave that advances in the circumferential direction of the vibration body 10. The n-th order driving vibration is a bending vibration in which the number of waves in the circumferential direction of the vibration body 200 is n. The driving vibration generated in the piezoelectric element 250 drives the contact body 300 in the circumferential direction around the shaft by the traveling waves generated in contact parts 214 of the vibration body 200. That is, the contact body 300 rotates relative to the vibration body 200 while maintaining its concentricity with the vibration body 200. The rotational force generated in the contact body 300 is output to the outside through the pressure mechanism 40 and the shaft.

The vibration actuator 10 of the present embodiment illustrated in Fig. 1 can freely rotationally drive a movable object by fixing the housing to a desired member and fixing the movable object to the shaft, for example. On the other hand, the vibration actuator 10 can also rotationally drive the housing with the shaft fixed.

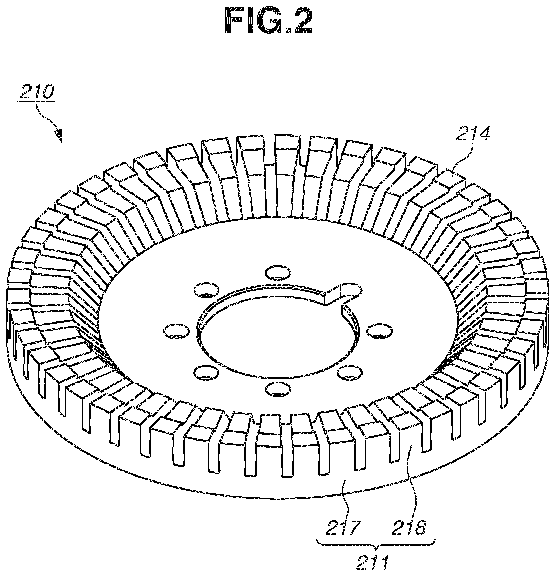

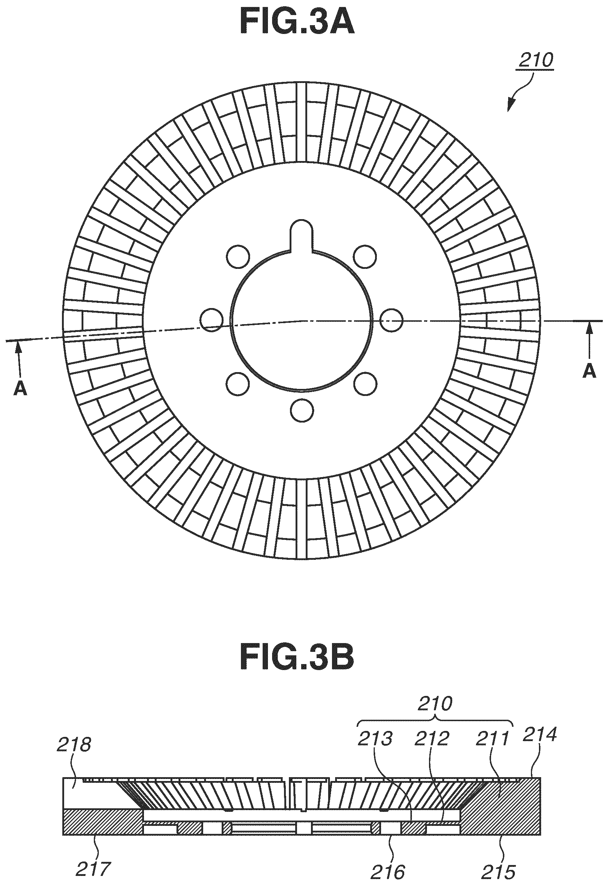

Fig. 2 is a schematic perspective view of a configuration of the elastic body 210. Fig. 3A is a schematic front view of a configuration of the elastic body 210, and Fig. 3B is a schematic cross-sectional view of a configuration of the elastic body 210. The elastic body 210 is integrally formed with a vibrating part 211, a connecting part 212, and an attachment part 213. The vibrating part 211 has a base part 217 and a plurality of protrusions 218.

The piezoelectric element 250 (not illustrated) is adhered or joined to an adhesion surface 215 (surface parallel to the radial direction), which is one surface of the base part 217. On the surface of the base part 217 opposite to the surface to which the piezoelectric element 22 is adhered, the plurality of protrusions 218 for amplifying vibration displacement is formed at substantially equal intervals on the same circumference so as to protrude in the thickness direction of the base part 218. A part of the tip surface of each protrusion 218 forms a contact surface 214 that comes into pressure contact with the contact body 300 (not illustrated). The elastic body 210 is fixed to a housing (not illustrated) equipped with a bearing at an attachment surface 216 of the attachment part 213.

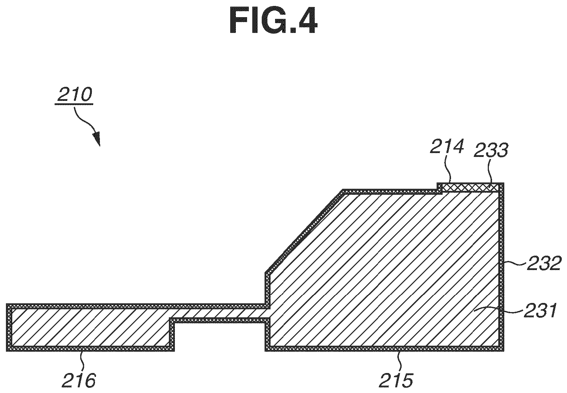

Fig. 4 is a schematic cross-sectional view of a configuration of materials of the elastic body 210. The elastic body 210 includes a base material 231, and an anti-nitriding layer 232 and a nitrided layer 233 that are formed on the surface of the base material 231. In Fig. 4, the thicknesses of the anti-nitriding layer 232 and the nitrided layer 233 are exaggerated. In reality, the size of the elastic body 210 is on the order of millimeters, the thickness of the anti-nitriding layer 232 is 1 µm to 100 µm, and the thickness of the nitrided layer is 10 µm to 100 µm. The anti-nitriding layer 232 is preferably 10 µm or less thick to prevent the occurrence of cracks on the surface during the manufacturing process. The "thickness" is defined as the average value of thicknesses at different points on the surface layer.

The base material 231 of the elastic body 210 is made of a non-magnetic steel material. The non-magnetic steel material is an austenitic steel material called austenitic stainless steel or austenitic heat-resistant steel.

The nitrided layer 233 with a thickness of 10 µm to 100 µm is formed on the surface including the contact surface 214 to the contact body 300 by nitriding the base material 231. The nitrided layer 233 is a layer in which nitrogen penetrates and diffuses into the base material 231 to partially form a nitrided compound of iron or chrome for the purpose of improving wear resistance, and has a higher nitrogen content (mass%) than the base material. The hardness of the outermost surface of the nitrided layer is HV1000 or more. The hardness decreases in the depth direction, becoming the same as the hardness of the base material 231 at a depth of 10 µm to 100 µm.

The anti-nitriding layer 232 is formed on the surface of the elastic body 200, not excluding the contact surfaces 214 to the contact body 300. The anti-nitriding layer 232 is obtained by forming an electroless nickel-phosphorus plating film with a phosphorus content (mass%) of 4% or more and 13% or less on the base material 231 and then nitriding the resultant. The anti-nitriding layer 232 is a layer in which the components of the base material, nickel, and phosphorus are interdiffused. The composition of the anti-nitriding layer 232 is characterized by higher nickel and phosphorus contents (mass%) than the base material. In addition, the composition of the anti-nitriding layer 232 is also characterized by a gradient composition in which the nickel and phosphorus contents decrease and the iron and chrome contents increase from the outermost surface in the depth direction. The phosphorus content of the plating film before the nitriding treatment is 4% or more and 13% or less, but the phosphorus content of the outermost surface decreases due to the diffusion of phosphorus in the depth direction and dephosphorization from the surface by the nitriding treatment. However, if a nitriding treatment condition is low temperature or short time, the phosphorus content will be hardly reduced, so the phosphorus content after the nitriding treatment may be considered to be 4% or more and 13% or less, which is the same as the content of the plating film. The relationship between the phosphorus content and the magnetic property will be described below.

The phosphorus content of the outermost surface the magnetism of the anti-nitriding layer 232 is smaller than that of the nitrided layer 233 and is larger than that of the base material 231.

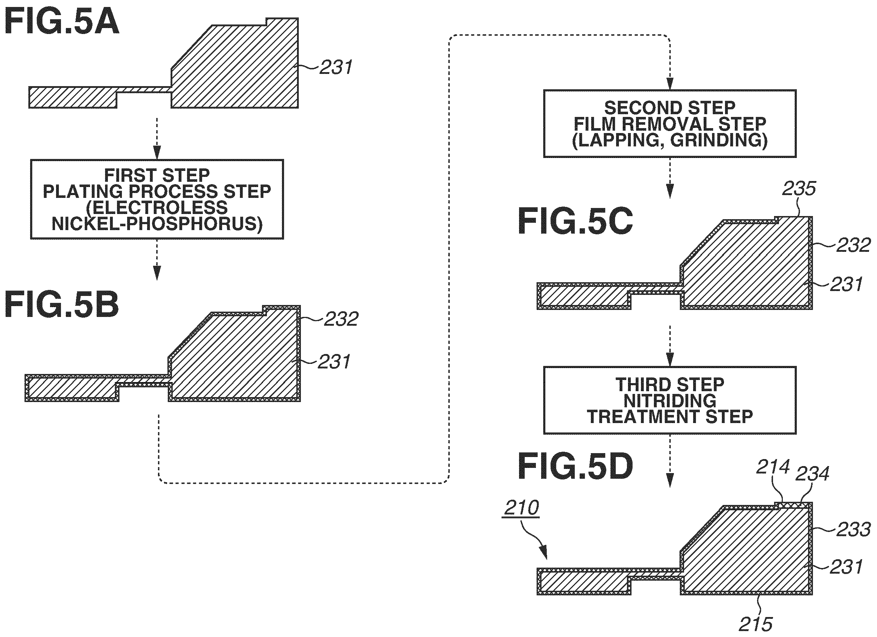

A manufacturing method of the elastic body of the present exemplary embodiment will be described. Fig. 5 is a schematic diagram illustrating a first manufacturing process of the elastic body 210. The elastic body 210 illustrated in Fig. 5A is made of an austenitic steel material as a base material, and is manufactured such that the vibrating part 211, the connecting part 212, and the attachment part 213 are integrally formed by cutting, forging, or the like, and the vibrating part 111 has the plurality of protrusions 218.

The first step is a plating process step for the base material 231. The entire surface of the base material 231 is covered with an electroless nickel-phosphorus film 232 with a thickness of 1 µm to 100 µm. That is, if the surface having higher nickel and phosphorus contents [mass%] than the base material is defined as a first surface, the thickness of the first surface is 100 µm or less, preferably 10 µm or less, and more preferably 5 µm or less.

The electroless nickel-phosphorus plating film 232 is generally of a type called a medium phosphorus to high phosphorus type, and its phosphorus content is preferably 7% or more and 13% or less (mass%), and more preferably 10% or more and 13% or less (mass%). Fig. 5B illustrates the elastic body after the first step.

The second step is a film removal step for removing the electroless nickel-phosphorus plating film 232 from the surface including the contact surfaces 214 to the contact body 300. In the second step, the electroless nickel-phosphorus plating film is removed by mechanical processing such as cutting, grinding, or polishing to expose an exposed surface 235 of the base material 231 which is a steel material. Fig. 5C illustrates the elastic body after the second step.

In the second step, it is preferable to improve the flatness by removing the electroless nickel-phosphorus plating film and grinding the exposed surface 235 of the steel material. This makes it possible to omit or simplify the polishing process for improving the flatness of the contact surfaces 214 after the nitriding treatment in the next third step.

The third step is a nitriding treatment step. In the nitriding treatment step, since the portion covered with the electroless nickel-phosphorus plating film 232 is made nitriding-resistant, a nitrided layer 234 is formed only on the exposed surface 235 from which the electroless nickel-phosphorus plating film has been removed. Accordingly, the highly wear-resistant nitrided layer 234 can be selectively formed in a narrow area that is required for pressure contact with the contact body 300. The nitriding treatment may be performed by salt bath nitriding, gas nitriding, ion nitriding, or other methods. However, since all of these methods involve treatment at high temperatures of about 400 to 600°C, the anti-nitriding layer 233 is formed in which the electroless nickel-phosphorus film 232 and the base material 231 are interdiffused. Fig. 5D illustrates the elastic body after the third step.

Although no particular step is necessary after the third step if the flatness of the surface of the nitrided layer 234 is good, it is desirable to provide a step of polishing the surface of the nitrided layer 234 so as not to thin the nitrided layer 234 as much as possible, in order to further improve the flatness of the surface of the nitrided layer 234. By keeping the amount of grinding of the nitrided layer 234 to a minimum, it is possible to maintain high wear resistance of the nitrided layer 234, which is the friction surface to the moving body 180, and therefore it is possible to suppress a decrease in the durability of the vibration actuator 10.

Subsequent to the fifth step, a step of removing the anti-nitriding layer 233 by polishing the adhesion surface 215 to which the piezoelectric element is adhered, or a step of roughening the surface of the adhesion surface 215 may be provided. This allows the elastic body 210 and the piezoelectric element 250 to be firmly adhered (or joined).

The selection of the plating process and plating film used in the first step will be described. If the purpose of the processing is to demagnetize the elastic body 210, it is considered preferable to use copper electroplating or chrome electroplating, which produces a non-magnetic film, for the plating process in the first step. However, it must be considered that the plating film will undergo nitriding treatment in the third step. In the nitriding treatment, the plating film undergoes changes in material at high temperatures, changes due to interdiffusion with the base material, and changes due to the intrusion of nitrogen. Accordingly, in order to demagnetize the elastic body, it is necessary to consider the magnetic property of the plating film after nitriding treatment. Therefore, in order to select a plating film suitable for demagnetization, the inventor of the present application performed various plating processes on SUS316, performed nitriding treatment, and evaluated the magnetic properties of the plating film and the base material.

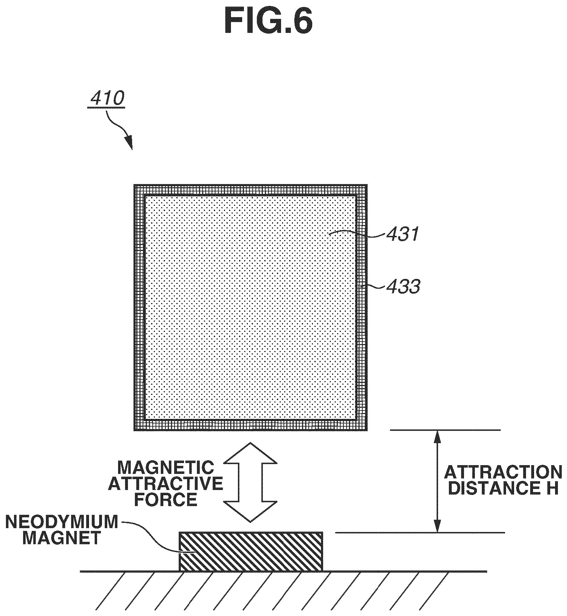

Fig. 6 is a schematic diagram illustrating a method for evaluating the magnetic property of a material. The base material of a test piece 410 is austenitic stainless steel (SUS316), and its entire surface is covered with various plating films and then subjected to nitriding treatment. The test piece 410 is gradually brought closer to a neodymium magnet (300 mT) placed on a stage from above, and the distance at which the neodymium magnet leaves the stage and floats due to magnetic attraction and is attracted to the test piece 410, is measured and defined as an attraction distance H. That is, the shorter the attraction distance H, the less magnetic the material is, and the longer the attraction distance H, the more magnetic the material is. In order to achieve the demagnetization of the elastic body 210 of the vibration actuator 10, it is desirable to use a material with a short attraction distance H.

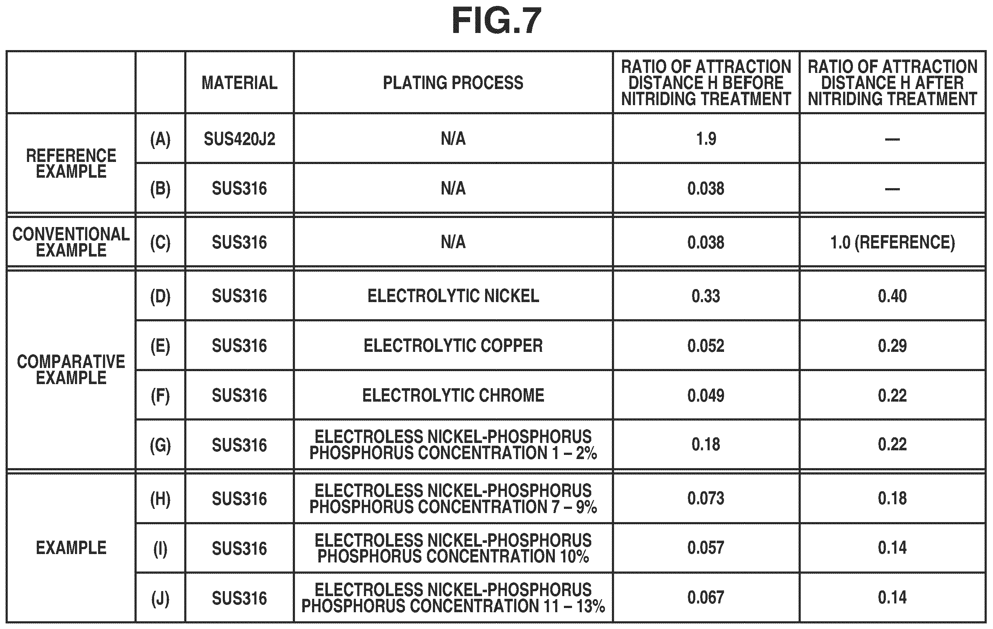

Fig. 7 is a table illustrating the evaluation results of magnetic properties of materials. In Conventional Example (C), as a non-magnetic material, austenitic stainless steel SUS316, was not subjected to plating but was subjected to nitriding treatment. The magnitude of magnetism was expressed as a ratio of the attraction distance H to the attraction distance H of (C) after nitriding treatment taken as 1.0. Higher ratios of the attraction distance H indicate greater magnetism, and lower ratios of the attraction distance H indicate smaller magnetism.

Fig. 7 illustrates, as reference examples (A) and (B), the evaluation results of magnetic properties of materials that were neither subjected to plating process nor nitriding treatment. Reference Example (A) is martensitic stainless steel SUS420J2, and Reference Example (B) is austenitic stainless steel SUS316. The ferromagnetic material SUS420J2 has a high attraction distance H ratio of 1.9, while the non-magnetic material SUS316 has a low attraction distance H ratio of 0.038.

Fig. 7 illustrates Conventional Example (C) in which austenitic stainless steel SUS316 was not subjected to a plating process but was subjected to nitriding treatment. The ratio of the attraction distance H was 0.038 before nitriding treatment and 1.0 after nitriding treatment. The ratio of the attraction distance H between before and after nitriding treatment is 1/0.038 = 26 times, which indicates that a nitrided layer was formed on the surface by nitriding SUS316, thereby increasing magnetism.

The following describes the evaluation results of magnetic properties of materials that were subjected to various plating processes and then nitriding treatment, with the base material being austenitic stainless steel SUS316. The thickness of the film formed by each plating process was 3 to 5 µm, and nickel strike plating with a thickness of 1 µm or less was applied as the base as necessary to ensure adhesion.

Fig. 7 illustrates Comparative Examples (D), (E), (F), and (G). The types of plating processes were nickel electroplating in (D), copper electroplating in (E), chrome electroplating in (F), and electroless nickel-phosphorus plating (phosphorus content of 1 to 2%) in (G).

The ratio of the attraction distance H before nitriding treatment (after plating) was 0.33 in (D) with nickel electroplating and was 0.18 in (G) with electroless nickel-phosphorus plating (phosphorus content of 1 to 2%), which were one order of magnitude greater than 0.038 of the SUS316 base material. This indicates that the plating film is magnetic because the main component, nickel, of the plating film is a ferromagnetic material.

The ratio of the attraction distance H after nitriding treatment was 0.40 in Comparative Example (D) and was 0.22 in Comparative Example (G), which were slightly increased from those before nitriding treatment. This indicates that, during the nitriding treatment, the plating films underwent any one or a combination of changes in the material properties at high temperatures, changes due to interdiffusion with the base material, and changes due to the intrusion of nitrogen, resulting in increased magnetism.

The ratio of the attraction distance H before nitriding (after plating) was 0.052 in (E) with copper electroplating and was 0.049 (F) with chrome electroplating, which were the same order of magnitude as 0.038 of the SUS316 base material. This indicates that the plating films are extremely low magnetism because the main components of the plating films, copper and chrome, are non-magnetic. The reason for their slightly higher magnetism than the base material is thought to be the magnetism of the underlying nickel strike plating. Although the plating films has low magnetism, the ratio of attraction distance H after nitriding treatment was 0.29 in (D) and was 0.52 in (G), which increased one order of magnitude. This indicates that the plating films had magnetism increased during the nitriding treatment due to any one or a combination of changes in the material properties at high temperatures, changes due to interdiffusion with the base material, and changes due to the intrusion of nitrogen.

In the above four comparative examples, the ratios of the attraction distance H after nitriding treatment were 0.22 to 0.40 times that of Conventional Example (C), with reduction in magnetism. However, none of the plating films is sufficient to achieve the demagnetization of the elastic body 210 of the vibration actuator 10.

Fig. 7 illustrates Examples (H), (I), and (J). In all the examples, the type of plating was electroless nickel-phosphorus plating. The phosphorus content of the plating film before nitriding was 7 to 9% in (H), 10% in (I), and 11 to 13% in (J). Since electroless nickel has low adhesion to SUS316, nickel strike plating with a thickness of 1 µm or less was applied as the base to improve adhesion in all the examples. The ratio of the attraction distance before nitriding treatment was 0.073 in (H), 0.057 in (I), and 0.067 in (J), which were sufficiently low and the same order as the base material SUS316. The inventor of the present application expected that the magnetism of the electroless nickel phosphorus plating would decrease as the phosphorus content increased, but this tendency was not observed in this evaluation. It is presumed that the magnetism of the test pieces was very small, and was buried in the variation of the test pieces and measurement error, so that the tendency was not observed in (I) and (J). The ratio of the attraction distance H after nitriding treatment was 0.18 in (H), 0.14 in (I), and 0.14 in (J), and there was a tendency that the ratio of the attraction distance H was lower as the phosphorus content increased. In all the examples, the ratio of the attraction distance H after nitriding treatment was 0.14 to 0.18 times that of the conventional example (C), and the magnetism was small. Within a certain range of phosphorus content, the magnetism after nitriding treatment was small, so these examples are suitable as non-magnetic materials. Accordingly, it is possible to achieve both wear resistance and non-magnetism of the elastic body 210 of the vibration actuator 10.

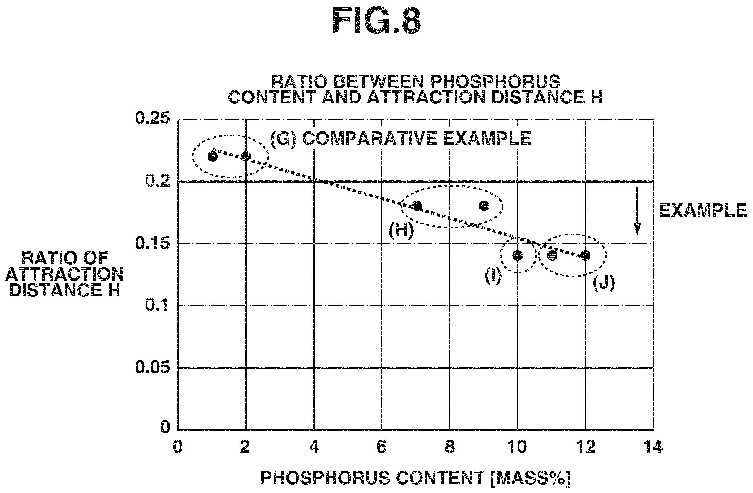

Fig. 8 illustrates a graph illustrating the evaluation results of magnetic properties of the materials. The horizontal axis indicates the phosphorus content (mass%) of the electroless nickel-phosphorus plating film before nitriding treatment, and the vertical axis indicates the ratio of the attraction distance H. The dotted line indicates a linear approximation equation using the least squares method. Along with the increase in the phosphorus content, the ratio of the attraction distance H decreases. Among the conventional examples, the ratio of the attraction distance H is the smallest, 0.22 in (F) with chrome electroplating and (G) with electroless nickel-phosphorus plating (phosphorus content of 1 to 2%). When the phosphorus content is 4% or more, the ratio of the attraction distance H is 0.20 or less, which is less magnetic than the conventional example, and therefore they are suitable as non-magnetic materials. Additionally, in this evaluation, the phosphorus content (mass%) was set to a maximum of 13%, but the higher the phosphorus content, the lower the magnetism of the electroless nickel-phosphorus plating film, so the magnetism after nitriding treatment is less than that of the conventional example.

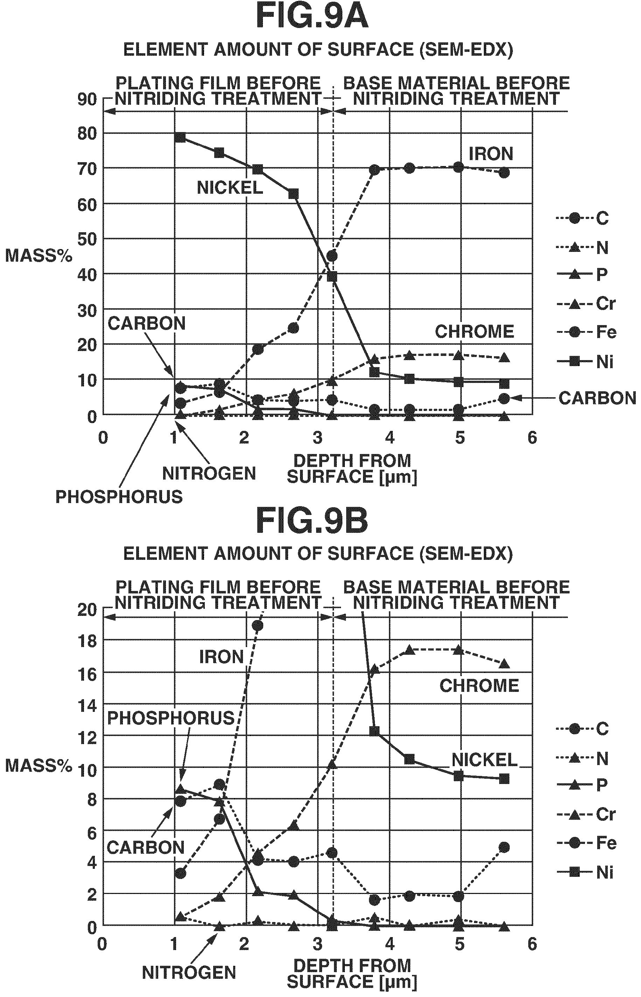

Fig. 9A is a graph of the amounts of elements in the cross section of the surface of the present exemplary embodiment. Fig. 9B is a graph in which the vertical axis of the graph in Fig. 9A is enlarged. The horizontal axis indicates the depth from the surface, and the vertical axis indicates the element content (mass%) analyzed by Scanning Electron Microscope-Energy Dispersive X-ray (SEM-EDX).

The test piece was electroless nickel-phosphorus plating (phosphorus content 11-13%) after nitriding treatment in (J). The thickness of the plating film before nitriding treatment was 3 to 4 µm including the underlying strike nickel plating film. Figs. 9A and 9B illustrate the approximate position of the interface between the plating film and the base material by dashed lines.

From the surface in the depth direction, the iron and chrome contents (mass%) gradually increased, while the nickel and phosphorus contents (mass%) gradually decreased. This is because iron and chrome, which were not included in the plating film before nitriding treatment, diffused from the base material to the surface, and nickel and phosphorus diffused from the plating film to the base material. In addition, almost no nitrogen was detected from the surface or the base material, which indicates that the electroless nickel plating film (phosphorus content of 11 to 13%) functioned as an anti-nitriding layer. It can be seen that the element composition was constant in the region deeper than 4 to 5 µm from the surface, and this was the composition amount of the base material SUS316, which was not affected by the nitriding treatment. Although there is a quantitative difference between the composition of the base material and that of general SUS316, it is sufficient for a qualitative understanding.

In the present exemplary embodiment, the manufacturing process of the elastic plate 210 has been described as a manufacturing process in which the plating film is removed from the surface that will come into contact with the contact body 300 after the plating process, but the present invention is not limited to this. The surface that will come into contact with the contact body 300 may be covered with a mask member during the plating process such that no plating film is formed on the surface. In this manufacturing process, the plating film removal step can be omitted.

In the present exemplary embodiment, the elastic body 210 of the vibration actuator 10 is taken, but the present invention is not limited thereto. The technology of the present exemplary embodiment may be applied to the contact body 300 that comes into pressure contact with the elastic body 210. The contact body 300 is formed of an austenitic steel material and subjected to electroless nickel plating (phosphorus content 4 to 13%), then the plating film is removed from the contact surface, and then the contact body 300 is subjected to nitriding treatment, thereby achieving both wear resistance and non-magnetism. The technology of the present exemplary embodiment may be applied to both the elastic body 210 and the contact body 300.

### Second Exemplary Embodiment

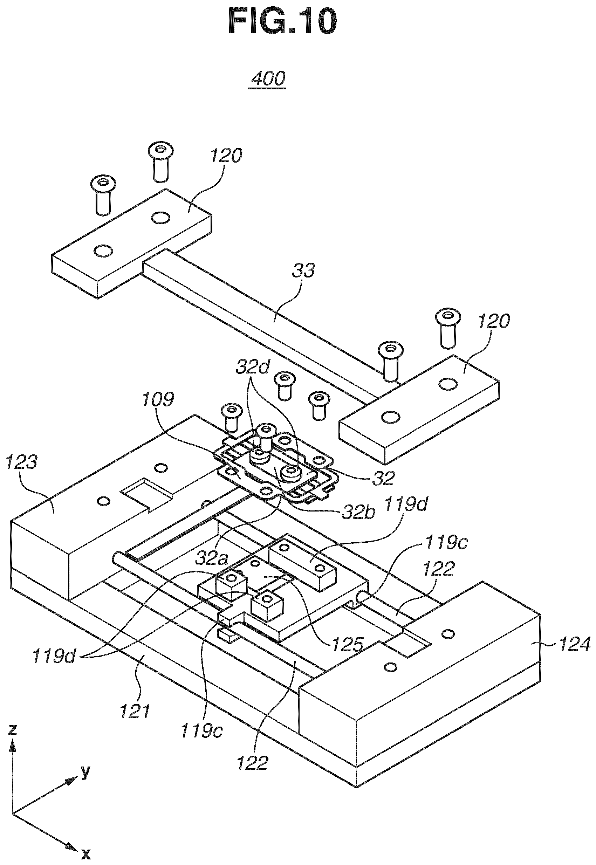

Fig. 10 is a schematic exploded perspective view of a configuration of a vibration actuator according to a second exemplary embodiment of the present invention. A vibration actuator 400 illustrated in Fig. 10 is a device capable of linear driving, and includes a vibration body 32 and a contact body 33 that move relative to each other.

The vibration body 32 has an elastic body 32b, two protrusions 32d provided on one surface of the elastic body 32b, and a piezoelectric element 32a provided on the surface opposite to the surface on which the protrusions 32d are provided. The contact body 33 can be driven with at least one protrusion 32d.

The piezoelectric element 32a is adhered to the elastic body 32b by an adhesive. The piezoelectric element 32a has a structure in which electrodes of a predetermined shape are formed on both sides of a plate-shaped piezoelectric ceramic. A driving voltage (alternating-current voltage) of a predetermined frequency is applied to the electrodes of the piezoelectric element 32a to excite the vibration of the vibration body 32 in two vibration modes, and the protrusions 32d are caused to make elliptical motion in a plane including the direction connecting the two protrusions 32d and the protruding direction of the protrusions 32d. Accordingly, the protrusions 32d frictionally drive the contact body 33, and the contact body 33 and the vibration body 32 can be linearly driven relative to each other. The principle of causing the vibration body 32 to make elliptical motion is well known, so detailed description thereof will be omitted herein.

The vibration body 32 is held by and fixed to a support member 119. The support member 119 is guided by two guide bars 122, which are guide members arranged parallel to the X direction in Fig. 6, and is movable in the X direction. The two guide bars 122 are fixed in a state of being sandwiched between two driven body holding members 123 and 124 fixed to a bottom plate 121.

The vibration body 32 is screwed to fixing parts 119d provided on the support member 119 via a holding member 109. This allows the support member 119 and the vibration body 32 to move integrally in the X direction.

There is interposed a pressure spring 125 between the support member 119 and the vibration body 32. The pressure spring 125 is a pressure means that has a leaf spring shape and generates a spring force by bending in the Z direction. The free end of the pressure spring 125 is in contact with the vibration body 32 to press the vibration body 32 against the contact body 33. In order to achieve a desired pressure contact state, the vibration body 32 and the contact body 33 need to be relatively displaceable in the Z direction. **In** the present exemplary embodiment, the vibration body 32 and the contact body 33 are relatively displaceable in the Z direction by a part of the holding member 109 being elastically deformed.

In the vibration actuator 400, the support member 119 that slides in the X direction together with the vibration body 32 is used as output means to extract a driving force (or displacement) for driving any device.

The vibration actuator 400 is mainly made of a non-magnetic material such as austenitic steel, brass, phosphor bronze, aluminum alloy, or ceramic.

The technology of the first exemplary embodiment can be applied to the elastic body 32b or the contact body 33 of the second exemplary embodiment. The elastic body 32b or the contact body 33 is made of an austenitic steel material and subjected to electroless nickel plating (phosphorus content of 7 to 13%), then the plating film is removed from the contact surface, and then the elastic body 32b or the contact body 33 is subjected to nitriding treatment, thereby achieving both wear resistance and non-magnetism. The technology of the first exemplary embodiment may be applied to both the elastic body 32b and the contact body 33.

### Third Exemplary Embodiment

In a third exemplary embodiment, a biopsy puncture device will be taken in which the rotary shaft of the rotary vibration actuator of the first exemplary embodiment made of a non-magnetic material is coupled to a needle. An example in which the puncture device is applied to a magnetic resonance imaging (MRI) diagnostic apparatus, which is a medical system, will be described.

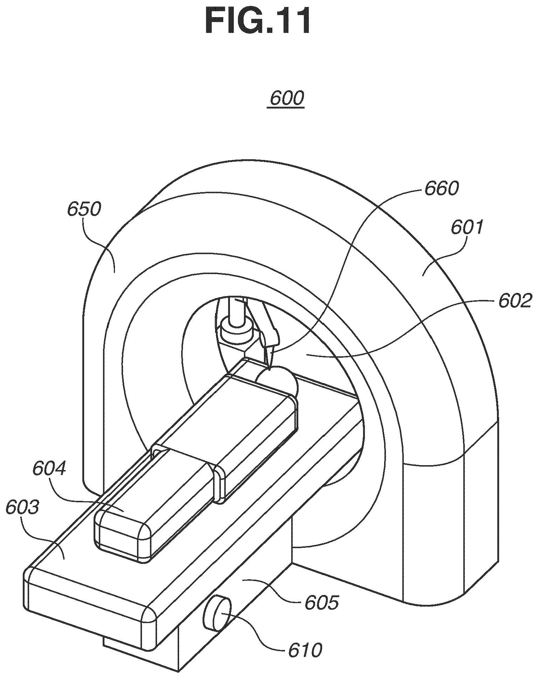

Fig. 11 is a schematic perspective view of a configuration of an MRI diagnostic apparatus 600 equipped with a puncture device 660.

The MRI diagnostic apparatus 600 generates a strong magnetic field. Thus, in the case of installing devices using magnetic materials, such as electromagnetic motors that use magnets, it is necessary to take preventive measures so as not to exert any influence on diagnostic images. In contrast, the vibration actuator does not use a magnet, and the vibration body and the contact body are made of austenitic steel material, so that the vibration actuator can be installed near or inside the MRI diagnostic apparatus 600.

While the MRI diagnostic apparatus 600 acquires image information of a subject, the needle attached to the leading end of the puncture device 660 extracts a living body tissue.

Specifically, the puncture device 660 is arranged near the doughnut-shaped MRI diagnostic apparatus 600. The MRI diagnostic apparatus 600 has a magnetic field generation unit 601 formed in a ring shape, a bore 602 that is a cylindrical portion into which a subject enters, a treatment table 603 on which a subject 604 lies, and a support table 605 that moves the treatment table 603 closer to/away from the bore 602. The puncture device 660 is arranged at a position where the needle can be inserted into the bore 602.

Based on the image information acquired by the MRI diagnostic apparatus 600, the puncture device 660 is driven to perform biological extraction using the needle.

The support table 605 is also equipped with a vibration actuator as a drive source for moving the treatment table 603. As described above, the vibration actuator does not use a magnet, and thus can be arranged in the vicinity of the magnetic field generation unit 601. This makes it possible to increase the degree of freedom in designing the treatment table 603.

In the above example, the needle is inserted into the bore 602 of the doughnut-shaped MRI diagnostic apparatus 600. However, the configuration of the MRI diagnostic apparatus is not limited to the doughnut shape.

Although the present invention has been described in detail based on the preferred exemplary embodiments, the present invention is not limited to these specific exemplary embodiments, and various forms within the scope of the gist of the present invention are also included in the present invention. Further, each of the above-described exemplary embodiments is merely one exemplary embodiment of the present invention, and the exemplary embodiments can be combined as appropriate.

That is, the present invention can be suitably applied to medical devices including a member and the above-described vibration actuator that is provided on the member and operates under a strong magnetic field environment.

As another example, the present invention can be suitably applied to electronic devices including a member and the above-described vibration actuator provided on the member.

The present invention is not limited to the above-described exemplary embodiments, and various modifications and variations can be made without departing from the spirit and scope of the present invention. Therefore, the following claims are appended to apprise the public of the scope of the present invention.

This application claims priority based on Japanese Patent Application No. 2022-190041 filed on November 29, 2022, the entire contents of which are incorporated herein by reference.

### Description of Reference Signs

- 10: Vibration Actuator
- 200: Vibration body
- 210: Elastic body
- 250: Piezoelectric element
- 50: Power supply member
- 300: Contact body
- 214: Contact surface
- 215: Adhesion surface
- 216: Attachment surface
- 231: Base material
- 232: Anti-nitriding layer
- 233: Nitrided layer

## Claims

1. A vibration actuator comprising:
a vibration body having an electro-mechanical energy conversion element and an elastic body; and
a contact body in contact with the elastic body,
wherein the vibration body and the contact body move relatively to each other due to vibration of the vibration body,
wherein at least one of the elastic body and the contact body has a base material made of an austenitic steel material,
wherein a portion of a surface of the base material includes a first surface having higher contents [mass%] of nickel and phosphorus than a part of the base material other than the surface, and a second surface having a higher content [mass%] of nitrogen than the part of the base material other than the surface, and
wherein the elastic body and the contact body are in contact with each other on the second surface.

2. The vibration actuator according to Claim 1, wherein the contents [mass%] of nickel and phosphorus decrease and contents [mass%] of iron and chrome increase from the first surface in a depth direction.

3. The vibration actuator according to Claim 1 or 2, wherein magnetism of the second surface is smaller than magnetism of the first surface, and is larger than magnetism of the base material.

4. The vibration actuator according to Claim 1 or 2, wherein the first surface has a thickness of 100 µm or less.

5. The vibration actuator according to Claim 4, wherein the first surface has a thickness of 10 µm or less.

6. The vibration actuator according to Claim 5, wherein the first surface has a thickness of 5 µm or less.

7. The vibration actuator according to Claim 1 or 2, wherein a phosphorus content [mass%] of the first surface layer is 4% or more and 13% or less.

8. The vibration actuator according to Claim 7, wherein the phosphorus content [mass%] of the first surface layer is 7% or more and 13% or less.

9. The vibration actuator according to Claim 8, wherein the phosphorus content [mass%] of the first surface layer is 10% or more and 13% or less.

10. A medical device comprising:
a member; and
the vibration actuator according to Claim 1 or 2, which is provided on the member and operates under a strong magnetic field environment.

11. An electronic device comprising:
a member; and
the vibration actuator according to Claim 1 or 2, which is provided on the member.

12. A manufacturing method of a vibration actuator in which a vibration body having an electro-mechanical energy conversion element and an elastic body is excited to vibrate, thereby causing relative movement between the vibration body and a contact body in contact with the elastic body, the manufacturing method comprising:
forming an electroless nickel-phosphorus plating film with a phosphorus content [mass%] of 4% or more on a surface of a base material of at least one of the elastic body and the contact body, the base material being made of an austenitic steel material; and
after the forming the plating film, forming a nitrided layer on a contact surface of the elastic body that comes into contact with the driven body by subjecting the contact surface to nitriding treatment.

13. The manufacturing method of a vibration actuator according to Claim 12, further comprising:
after the forming the plating film, removing a portion of the electroless nickel-phosphorus plating film; and
forming a nitrided layer on a surface from which the plating film has been removed.

14. The manufacturing method of a vibration actuator according to Claim 12 or 13, wherein the electroless nickel-phosphorus plating film has a thickness of 100 µm or less.

15. The manufacturing method of a vibration actuator according to Claim 14, wherein the electroless nickel-phosphorus plating film has a thickness of 10 µm or less.

16. The manufacturing method of a vibration actuator according to Claim 15, wherein the electroless nickel-phosphorus plating film has a thickness of 5 µm or less.

17. The manufacturing method of a vibration actuator according to Claim 12 or 13, wherein the phosphorus content [mass%] of the plating film is 4% or more and 13% or less.

18. The manufacturing method of a vibration actuator according to Claim 17, wherein the phosphorus content [mass%] of the plating film is 7% or more and 13% or less.

19. The manufacturing method of a vibration actuator according to Claim 18, wherein the phosphorus content [mass%] of the plating film is 10% or more and 13% or less.
